# EUROPEAN PATENT SPECIFICATION

(11) **EP 0 668 769 B1**
(45) Date of publication and mention of the grant of the patent: **19.04.2000**
(21) Application number: 94900557.3
(22) Date of filing: 04.11.1993
(51) Int. Cl.: A61K 35/78, A61K 33/00

(54) **SYNTHETIC COMPOSITIONS RELATED TO OAK BARK EXTRAKT, AND METHOD OF USING SAME**
EICHENRINDENEXTRAKTVERWANDTE SYNTHETISCHE ZUBEREITUNGEN UND VERFAHREN ZUR VERWENDUNG
COMPOSITIONS SYNTHETIQUES ASSOCIEES A L'EXTRAIT D'ECORSE DE CHENE, ET PROCEDE D'UTILISATION DE CES COMPOSITIONS

(30) Priority: 06.11.1992 US 973071
(43) Date of publication of application: 30.08.1995
(73) Proprietor: GREYSTONE MEDICAL GROUP, INC., Memphis, Tennessee 38111 (US)
(72) Inventor: HON, David, N., S., Clemson, SC 29631 (US); STANLEY, R., Thomas, Auburndale, FL 33823 (US)
(74) Representative: Lally, William
(86) International application number: PCT/US93/10670
(87) International publication number: WO 94/11010

(56) References cited:
- EP-A- 0 217 975
- DE-C- 812 341
- DE-C- 819 127
- DE-C- 833 840
- US-A- 3 928 584
- US-A- 5 080 900

## Description

This application relates to synthetic compositions related to aqueous oak bark extract, synthetic containing the key active ingredients of oak bark extract and to the use of such compositions in the manufacture of a medicament for the treatment of skin cancer and other skin disorders; the application does not relate to compositions comprising an aqueous extract of oak bark ash as obtainable by burning oak bark to ash, mixing the screened, cooled ash with water to obtain a slurry, boiling the slurry and filtering the residue to obtain the extract as filtrate.

EP-A-0217975 discloses an inorganic salt mixture for the treatment of psoriasis. The mixture is composed primarily of a magnesium halide such as magnesium chloride, with mixed alkaline earth metal salts. Other cations present in the mixture may include strontium, aluminium, iron, lithium and zinc. Other anions include sulphate, hydrogen carbonate, borate, fluoride, silicate, iodide and carbonate.

Oak bark extract has been described in United States Patent No. 5,080,900 for use in the treatment of skin ulcers, particularly decubitus ulcers or bed sores. This material in a base of Whitfield's ointment has also been sold under the trade name Bencelok® for use in the treatment of minor skin irritations. The amount of oak bark extract in these materials was relatively low, however. For example, the Bencelok® preparations have continued from 0.25 to 3% by weight of ash-derived components based upon the total weight of the preparation.

It has now been found that higher concentrations of oak bark extract possess highly useful properties for the treatment of skin cancers, and that lower concentrations of oak bark extract possess additional therapeutic properties not heretofore recognised. For example, preparations containing 40-80% oak bark extract are useful in the treatment of acute cancerous skin ulcers. In addition, it has now been found that synthetic mixtures containing potassium ions, zinc ions, calcium ions provide many of the same advantageous properties of oak bark extract, and that the inclusion of rubidium ions and sulfate ions is advantageous.

Oak bark extract is prepared from oak bark ash. The bark utilised can be from Red Oak (Quercus rupra L), Black Oak (Quercus velutina Lam.), Shumerd Oak (Quercus shumardi i Buckl.), Scarlet Oak (Quercus coccinea Muenchb.), Willow Oak (Quercus phellos L.) and other species of the Erythrobalanus group. The oak bark is burned to convert it into an ash, which is cooled and screened to provide a powder.

The ash powder is then poured slowly into boiling water and boiled, with stirring, for a period of time (1.5 to 4 hours) to achieve an intermediate oak bark extract. The hot intermediate extract is then filtered to recover a clear filtrate and boiled for an additional period of time to achieve the desired final concentration of oak bark extract. During this boiling step, a white precipitate forms which is separated from the oak bark extract and discarded. Table 1 shows processing conditions which can be used to prepare oak bark extract of various final concentrations. The solution concentrations are expressed as weight percent of oak bark ash derived material.

**Table I**

| **Solution (%)** | **Temperature (°C)** | **Processing Time (Hours)** |
|---|---|---|
| 0.25 | 98±2 | 1.00 |
| 1.00 | 98±2 | 2.00 |
| 10.00 | 98±2 | 8.00 |
| 20.50 | 98±2 | 12.00 |
| 40.00 | 98±2 | 18.00 |
| 80.00 | 98±2 | 21.00 |

The oak bark extracts are complex mixtures of inorganic materials. Further, as is evident from the results of elemental analysis on the various solutions, (See Table 2) the relative amounts of the constituents vary from one concentration to another. For example, the 40% solutions (i.e., a solutions containing a total of 40% by weight of extracted oak bark materials and 60% by weight water) was found to be highly enriched in rubidium relative to lower concentration solutions.

The therapeutic activity of various constituents of oak bark extract has been analyzed with the result that silicon, strontium, barium, manganese, gallium, zirconium and titanium appear to be unnecessary, while therapeutic efficacy has been found for compositions containing just potassium, zinc and calcium ions, in combination with suitable counterions.

Thus, in accordance with the invention, there is provided a synthetic formulation containing, by weight of inorganic solids, 10 to 80 parts potassium ions, preferably 30 to 50 parts, 0.00001 to 20 parts zinc ions, preferably 1 to 10 pans, 0.01 to 10 parts calcium ions, preferably 1 to 5 parts, 1 to 40 parts rubidium ions, preferably 1 to 30 parts, and 0 to 5 parts sulfur, in the form of elemental sulfur or sulfate, together with pharmaceutically acceptable counterions (e.g., Cl⁻, SO₄⁼, CO₃⁼, OH⁻, Br⁻). The solution may also contain other inorganic cations, for example, up to 10 parts by weight of inorganic solids of cobalt, copper, iron, manganese, nickel, strontium or aluminium ions, preferably up to 1 part by weight. Further, the composition may include a pharmaceutically acceptable carrier such a water or an ointment or cream base which will result in a therapeutic composition having a pH of from 4 to 7, preferably pH 4.5 to 5.5.

The synthetic mixtures of the invention have been found to provide a variety of beneficial therapeutic properties. The therapeutic applications and the concentration of synthetic mixture related to oak bark extract by weight of solids are summarized in Table 3.

In particular, compositions containing about 20% or more, preferably 30% to 80% and more preferably 40% to 80%, of a similarly concentrated synthetic mixture to oak bark extract according to the invention can be used to treat cancerous and precancerous skin lesions. As used herein, the term cancerous and precancerous skin lesions includes but is not limited to basal cell epithelioma, squamous cell carcinoma, keratoacanthoma.

Compositions according to the invention are also useful for treating abrasions and other partial thickness wounds. Useful compositions include at least potassium, zinc and calcium ions and may include other ionic components as well as described in Examples 1 and 2. The composition is advantageously applied in a cream or ointment base over a period of several days. Similar compositions were found to be useful in the treatment of gangrene, impetigo, psoriasis, although longer periods of treatment may be required.

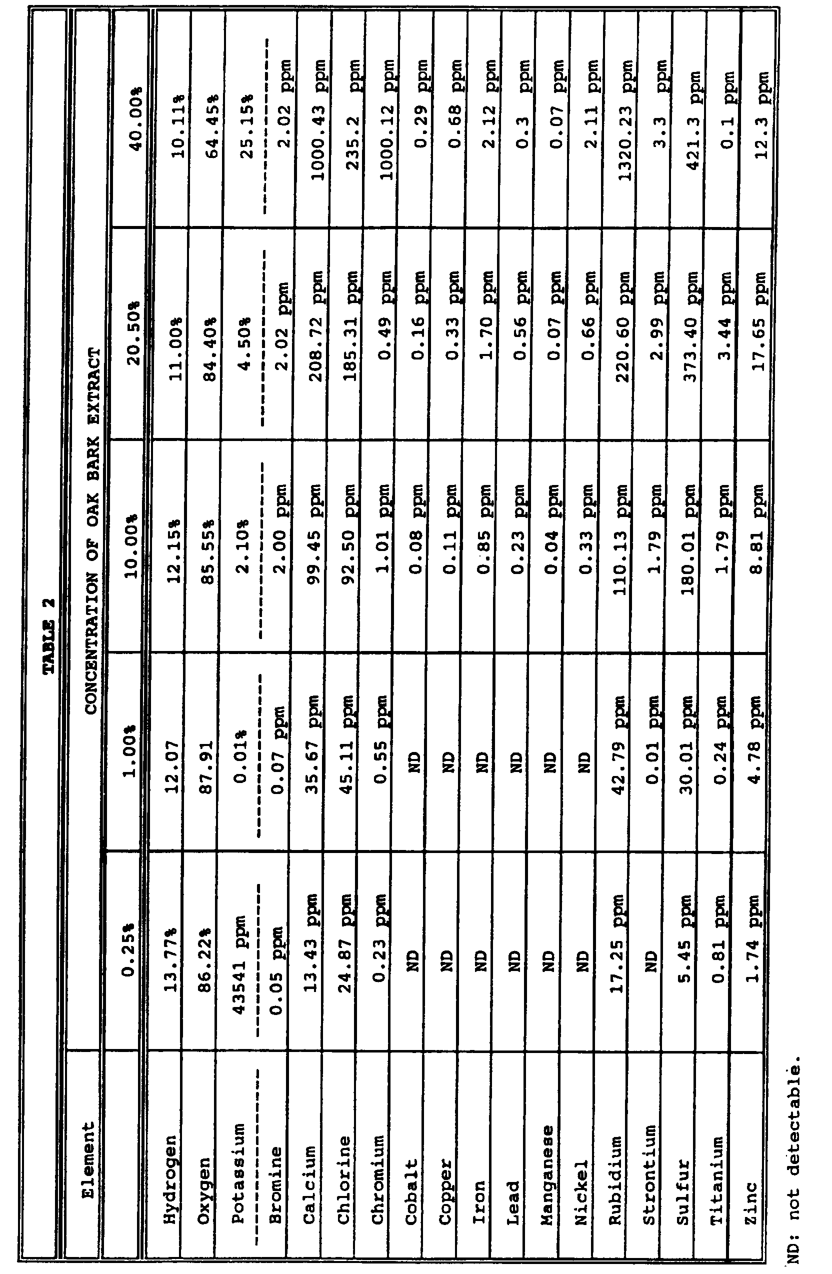

**Table III**

| Weight % of Oak Bark Extract | Indications |
|---|---|
| 0.25% | Fungal infection, minor infection, insect bites |
| 1.00% | Eczema, minor burns, sunburn, poison oak, poison ivy, poison sumac, wound healing |
| 3.00% | Pyodermas, dermatitis, pruritic dermatoses, eczema, minor burns, sunburn, poison oak, poison ivy, poison sumac, decubitus ulcers, tropical ulcers, wound healing |
| 5.00% | Decubitus, psoriasis |
| 10.00% | Psoriasis, impetigo, Kaposi sarcoma, warts, gangrene, ischemic ulcer, keratosis |
| 20.50% | Precancerous lesions, basal cell epithelioma, squamous cell carcinoma, keratoacanthoma |
| 40.00% | Acute cancerous ulcers |
| 80.00% | Acute cancerous ulcers |

While not intending to be bound by any particular mechanism of action, it appears that synthetic mixtures related to oak bark extract and containing the key ingredients of oak bark extract function to enhance wound healing by providing complexing ions which interact with enzymes such as alkaline phosphatase, carbonic anhydrase, carboxypeptidase, various enhydrogenases, arginase, carnosinase, dehydropeptidase, glycine dipeptidase, histidine deaminase and tripeptidase, oxyloacetic carboxylase, and some lecithinases and enolases. These enzymes are involved in numerous biosynthetic pathways necessary for wound healing, for example, collagen biosynthesis, and are believed to function with greater efficiency in the presence of the complexing ions.

The application will now be further described by way of the following, non-limiting examples.

### Example 1

A synthetic mixture was prepared by combining potassium sulfate (7.50 g), potassium hydroxide (7.65 g), calcium hydroxide (0.05 g), iron sulfate (0.4 mg), cobaltous bromide (0.1 mg), copper chloride (0.3 mg), zinc sulfate (1.2 mg), strontium chloride (0.3 mg) and rubidium sulfate (0.13 mg) in distilled water (59.09 g) and Whitfield's ointment (433.57 g). The total amount of synthetic chemicals was 3% in weight. The ointment was applied twice daily to three patients with severe abrasion. Prior to treatment, abrasions were cleaned with rubbing alcohol. All patients showed disappearance of abrasion within five days of treatments.

### Example 2

A synthetic mixture was prepared by combining zinc oxide (2.5 g), calcium hydroxide (2 g), potassium carbonate (3.5 g) and potassium hydroxide (12 g) in distilled water (80 g) and Whitfield's ointment (57 g). The total amount of synthetic chemicals was 3% in weight. This formulation was used to treat an outpatient with a severe abrasion on his left arm. The abrasion was cleaned with rubbing alcohol to remove any contamination. The ointment was applied to abrasion twice daily. The abrasion healed within 72 hours.

### Example 3

A synthetic mixture was prepared by combining zinc sulfate (2.5 g), calcium hydroxide (2 g), potassium carbonate (3.5 g) and potassium hydroxide (12 g) in distilled water (80 g) and Whitfield's ointment (57 g). The total amount of synthetic chemicals was 3% in weight. This formulation was used to treat an outpatient with venous stasis. The venous stasis was cleaned with a 3% solution of hydrogen peroxide, and pad dried. The ointment was applied to venous stasis twice daily. Reduction in ulcer size 60-70% within 72 hours was observed. Complete granulation within 5 days for ulcers less than 2 cm; 7 days for ulcers less than 4 cm.

### Example 4

A synthetic mixture was prepared by combining zinc sulfate (2.5 g), calcium hydroxide (2 g), potassium carbonate (3.5 g) and rubidium hydroxide (6 g), potassium hydroxide (6 g) in distilled water (80 g) and Whitfield's ointment (57 g). The total amount of synthetic chemicals was 3% in weight. This formulation was used to treat an outpatient with laceration. The laceration was cleaned with rubbing alcohol and air dried. The ointment was applied to the laceration twice daily. The laceration reduced its redness with 4 hours and healed with 72 hours.

### Example 5

A synthetic mixture was prepared by combining potassium hydroxide (6.6 g), rubidium hydroxide (0.4 g), zinc sulfate (0.6 g), sulfur (2 g) and calcium hydroxide (0.1 g) in distilled water (14.55 g) and Whitfield's ointment (72.75 g). The total amount of synthetic chemicals was 10% in weight. This formulation was used to treat an outpatient with psoriasis in the right arm and right leg. The psoriasis was cleaned with rubbing alcohol to remove any contamination. The ointment was applied to abrasion twice daily. The psoriasis healed in six weeks.

### Example 6

A synthetic mixture was prepared by combining potassium hydroxide (6.6 g), rubidium hydroxide (0.4 g), zinc sulfate (0.6 g), sulfur (2 g) and calcium hydroxide (0.1 g) in distilled water (14.55 g) and Whitfield's ointment (72.75 g). The total amount of synthetic chemicals was 10% in weight. This formulation was used to treat an outpatient with impetigo at the back. The back was thoroughly cleaned with rubbing alcohol to remove any contamination. The ointment was applied to impetigo twice daily. The impetigo healed in four weeks.

### Example 7

A synthetic mixture was prepared by combining potassium hydroxide (6.6 g), rubidium hydroxide (0.4 g), zinc sulfate (0.6 g), sulfur (2 g) and calcium hydroxide (0.1 g) in distilled water (14.55 g) and Whitfield's ointment (72.75 g). The total amount of synthetic chemicals was 10% in weight. This formulation was used to treat an outpatient with gangrene in his feet. The gangrene was thoroughly cleaned with hydrogen peroxide to remove any contamination. The ointment was applied to gangrene twice daily. The gangrene healed in six weeks.

## Claims

1. A composition comprising:
10 to 80 parts potassium ions;
0.00001 to 20 parts zinc ions;
0.01 to 10 parts calcium ions; and
1 to 40 parts rubidium ions by weight of inorganic solids, provided that the composition does not comprise an aqueous extract of oak bark ash, as obtainable by burning oak bark to ash, mixing the screened, cooled ash with water to form a slurry, boiling the slurry and filtering the residue to obtain the extract as filtrate.

2. A composition according to Claim 1, comprising 1 to 30 parts rubidium ions.

3. A composition according to one of Claims 1 and 2 comprising a pharmaceutically acceptable carrier.

4. A method of preparing a composition comprising the steps of combining together
i) 10 to 80 parts potassium ions;
ii) 0.00001 to 20 parts zinc ions;
iii) 0.01 to 10 parts calcium ions; and
iv) 1 to 40 parts rubidium ions by weight of
inorganic solids.

5. A method according to Claim 4, wherein the rubidium ions are mixed in the proportion of 1 to 30 parts.

6. A method according to one of Claims 4 and 5 comprising mixing the constituents with a pharmaceutically acceptable carrier.

7. The invention according to one of claims 3 and 6 wherein the carrier is (a) water, or (b) a creme base.

8. The use of a composition in accordance with any one of claims 1 to 3 or made in accordance with any one of claims 4 to 7 in the manufacture of a medicament for treating cancerous and precancerous skin lesions.

9. The use of a composition in accordance with any one of claims 1 to 3 or made in accordance with any one of claims 4 to 7 in the manufacture of a medicament for treating psoriasis.

10. The use of a composition in accordance with any one of claims 1 to 3 or made in accordance with any one of claims 4 to 7 in the manufacture of a medicament for treating impetigo.

## Patentansprüche

1. Zusammensetzung, umfassend:
10 bis 80 Teile Kaliumionen;
0,00001 bis 20 Teile Zinkionen;
0,01 bis 10 Teile Kalziumionen; und
1 bis 40 Teile Rubidiumionen bezogen auf Gewichtsteile anorganischer Feststoffe, vorausgesetzt, daß die Zusammensetzung keinen wässrigen Extrakt von Eichenrindenasche umfaßt, wie er durch Verbrennen von Eichenrinde zu Asche, Vermischen der gesiebten, abgekühlten Asche mit Wasser zu einem Brei, Kochen des Breis und Filtern des Rests hergestellt werden kann, um den Extrakt als Filtrat herauszuziehen.

2. Zusammensetzung nach Patentanspruch 1, umfassend 1 bis 30 Teile Rubidiumionen.

3. Zusammensetzung nach einem der Ansprüche 1 und 2, umfassend einen pharmazeutisch akzeptablen Trägerstoff.

4. Verfahren zur Herstellung einer Zusammensetzung, umfassend die folgenden Schritte zum Vereinigen von
i) 10 bis 80 Teile Kaliumionen;
ii) 0,00001 bis 20 Teilen Zinkionen;
iii) 0,01 bis 10 Teilen Kalziumionen; und
iv) 1 bis 40 Teilen Rubidiumionen bezogen auf Gewichtsteile anorganischer Feststoffe.

5. Verfahren nach Patentanspruch 4, wobei die Rubidiumionen im Verhältnis von 1 bis 30 Teilen zugemischt werden.

6. Verfahren nach einem der Patentansprüche 4 und 5, umfassend das Vermischen der Bestandteile mit einem pharmazeutisch akzeptablen Trägerstoff.

7. Erfindung nach einem der Ansprüche 3 und 6, wobei der Träger a) Wasser oder b) eine Cremebasis ist.

8. Verwendung einer Zusammensetzung nach einem der Patentansprüche 1 bis 3 oder hergestellt nach einent der Patentansprüche 4 bis 7 bei der Herstellung eines Medikaments zur Behandlung von Hautveränderungen durch Krebs oder Krebsvorstadien.

9. Verwendung einer Zusammensetzung nach einem der Ansprüche 1 bis 3 oder hergestellt nach einem der Ansprüche 4 bis 7 bei der Herstellung eines Medikaments zur Behandlung von Psoriasis.

10. Verwendung einer Zusammensetzung nach einem der Ansprüche 1 bis 3 oder hergestellt nach einem der Ansprüche 4 bis 7 bei der Herstellung eines Medikaments zur Behandlung von Impetigo.

## Revendications

1. Composition comprenant :
10 à 80 parties d'ions potassium ;
0,00001 à 20 parties d'ions zinc ;
0,01 à 10 parties d'ions calcium ; et
1 à 40 parties d'ions rubidium, en poids d'extrait sec inorganique, à la condition que la composition ne contienne pas un extrait aqueux de cendre d'écorce de chêne, tel qu'obtenu par combustion d'écorce de chêne jusqu'à incinération, mélange des cendres tamisées et refroidies avec de l'eau pour former une suspension, ébullition de la suspension et filtration du résidu pour donner l'extrait sous forme d'un filtrat.

2. Composition selon la revendication 1, comprenant 1 à 30 parties d'ions rubidium.

3. Composition selon l'une des revendications 1 et 2, comprenant un excipient acceptable d'un point de vue pharmaceutique.

4. Procédé de préparation d'une composition, comprenant les étapes de combinaison, les uns aux autres :
i) de 10 à 80 parties d'ions potassium ;
ii) de 0,00001 à 20 parties d'ions zinc ;
iii) de 0,01 à 10 parties d'ions calcium ; et
iv) de 1 à 40 parties d'ions rubidium, en poids d'extrait sec inorganique.

5. Procédé selon la revendication 4, dans lequel les ions rubidium sont mélangés selon une proportion de 1 à 30 parties.

6. Procédé selon l'une des revendications 4 et 5, comprenant le mélange de constituants à un excipient acceptable d'un point de vue pharmaceutique.

7. L'invention selon l'une des revendications 3 à 6, dans laquelle l'excipient est (a) l'eau, ou (b) une base de crème.

8. Utilisation d'une composition selon l'une quelconque des revendications 1 à 3 ou préparée selon l'une quelconque des revendications 4 à 7, pour fabriquer un médicament destiné au traitement de lésions cancéreuses et précancéreuses de la peau.

9. Utilisation d'une composition selon l'une quelconque des revendications 1 à 3 ou préparée, selon l'une quelconque des revendications 4 à 7, pour fabriquer un médicament destiné au traitement du psoriasis.

10. Utilisation d'une composition selon l'une quelconque des revendications 1 à 3 ou préparée selon l'une quelconque des revendications 4 à 7, pour fabriquer un médicament destiné au traitement de l'impétigo.
